# EUROPEAN PATENT SPECIFICATION

(11) **EP 1 194 423 B1**
(45) Date of publication and mention of the grant of the patent: **08.01.2003**
(21) Application number: 00938356.3
(22) Date of filing: 25.05.2000
(51) Int. Cl.: C07D 305/14

(54) **PROCESS FOR EXTRACTION AND PURIFICATION OF PACLITAXEL FROM NATURAL SOURCES**
VERFAHREN ZUM EXTRAHIEREN UND REINIGUNG VON PACLITAXEL AUS NATÜRLICHER QUELLE
PROCEDE D'EXTRACTION ET DE PURIFICATION DE PACLITAXEL PROVENANT DE SOURCES NATURELLES

(30) Priority: 22.06.1999 CA 2275980; 22.02.2000 CA 2299149
(43) Date of publication of application: 10.04.2002
(73) Proprietor: Chaichem Pharmaceuticals International, Laval, Québec H7V 4A9 (CA)
(72) Inventor: BUI-KHAC, Trung, Montréal, Québec H3S 1N5 (CA); DUPUIS, Nicolas, Laval, Québec H7N 3W4 (CA)
(74) Representative: Cabinet Hirsch
(86) International application number: CA0000619
(87) International publication number: WO00078741

(56) References cited:
- WO-A-94/13827

## Description

### TECHNICAL FIELD OF THE INVENTION

The present invention relates to an easy and quick process for the extraction and purification of Paclitaxel from natural sources. In comparison to the prior processes known in the art, this process is particularly economical because it has a limited number of steps and a reduced amount of losses during the purification.

### PRIOR ART

The first work concerning Paclitaxel, also called Taxol™ or Pacitaxeline™, started in the United States in the 1960's, when the National Cancer Institute began a program for selecting plant extracts having activities against cancerous tumours, or anti-neoplasic activities.

From 1960 to 1981, more than 110,000 composites were extracted from 35,000 species of plants and were isolated and tested (Blume E.J. Natl. Cancer Inst. 1991; 83: 1054-1056).

Yew is part of the plants that were selected and tested, and the first extract from yew barks (*Taxus brevifolia Nutt*) coming from the west coast of the United States (Oregon) was obtained by Wani et al (Wani et al, J. Am. Chem. Soc., 1971; 93:2325-2327). The crude extract of these barks demonstrated a cytotoxic activity against leukaemic cells and an inhibiting action against a variety of tumours.

A couple of years later, the active compound of the extract was isolated. This active compound has been given Paclitaxel as generic name, and its molecular structure has been determined by X-ray crystallography and by 1H-NMR spectrum (Wani et al. J. Am. Chem. Soc. 1971; 93:2325-2327).

Since that time, studies on the effect of Paclitaxel towards cancerous tumours in-vitro and in-vivo have been carried out and the positive results that were obtained have lead to classify this active compound as one of the promising drugs for the treatment of ovarian cancer and breast cancer (Rowinski et al. Pharmacol. Ther. 1991; 52: 35-84). By the way, the use of Paclitaxel for the treatment of different cancers has been approved by the Food and Drug Administration since 1992.

The first variety of yew that was used to produce Paclitaxel, was *Taxus brevifolia,* but other varieties found in different regions of the earth have also been tested. They consist of *Taxus baccata, Taxus canadensis, Taxus wallichiana, Taxus yunnanensis, Taxus densiformis, Taxus hicksii, Taxus wardii, Taxus cuspidata, Taxus capitata, Taxus brownii* (Miller et al. J. Org. Chem. 1981; 46: 1469; McLaughlin et al. J. Nat. Prod. 1981; 44: 321; Kingston et al. J. Nat. Prod 1982; 45: 466; Senilh et al. J. Nat. Prod. 1984; 47: 131-137; Huang et al. J. Nat. Prod. 1986; 49: 665-669; Fett-Neto et al. Bio/Technology 1992; 10: 1572-1575).

All these species contain Paclitaxel, but in very limited amounts-about 0.0004 to 0.008%- (Kingston, Pharmacol. Ther. 1991; 52: 1-34). This low concentration of Paclitaxel makes its extraction and purification very costly because it takes time and it generally calls for repeated chromatographies.

The low concentration of Paclitaxel in all the varieties of yew produces a huge impact on the environment. To extract 1 kg of Paclitaxel from *Taxus brevifolia* barks, one needs to cut down around 3000 grown trees to get 10,000 kg of bark. The obtained quantity (1 kg) of Paclitaxel permits to treat about 500 patients but the number of patients suffering from cancer is as high as hundreds of thousands. Replanting the trees will never meet the urgent demand of Paclitaxel for human needs, because of their slow growth (Vidensek et al. J. Nat. Prod. 1990; 53: 1609-1610; Kelsey et al. J. Nat. Prod. 1992; 55: 912-917; Wheeler et al. J. Nat. Prod 1992; 55: 432-440).

Numerous processes for extracting and purifying Paclitaxel have been proposed. For example, Wani et al. (Wani et al. J. Am. Chem. Soc, 1971; 93: 2325-2327) have proposed a process for extraction of Paclitaxel from the barks of yew of the United States' west coast (*Taxus brevifolia*) comprising a treatment of the barks with alcohol followed by a few steps of purification by chromatography.

Miller et al. (1981) have extracted Paclitaxel from the *Taxus wallichiana Zucc by a* the following process:
1) extraction from the plant and concentration of the extract;
2) fat removal by separation of water and hexane;
3) extraction with chloroform and concentration;
4) first purification by chromatography in a first silica column;
5) second purification by chromatography in a second silica column;
6) a first countercurrent distribution;
7) a second countercurrent distribution;
8) preparative HPLC chromatography.

Senilh et al. (1984) have isolated Paclitaxel (or Taxol™ A: 0.0165%), Cephalomannine (or Taxol™ B: 0.0064%) and other compounds from barks of the *Taxus baccata* by the following process:
1) extraction with alcohol and concentration;
2) separation of water and dichloromethane;
3) filtration chromatography;
4) chromatography in a silica column;
5) alumina chromatography;
6) chromatography in a medium pressure silica column;
7) HPLC chromatography.

For other analog, two or three other column chromatography treatments followed by a preparative HPLC chromatography are necessary.

Another process used by Polysciences Inc. comprises the following steps:
1) dried ground barks are treated with methanol or ethanol and the obtained extract is concentrated to remove the alcohol,
2) the concentrate is then treated with dichloromethane and the obtained solvent extract is concentrated to yield a powder,
3) the powder is dissolved with a mixture of acetone and ligroin (1:1) and filtered to remove insoluble matter,
4) the organic phase which contains Paclitaxel is concentrated, dissolved in 30% of ligroin, and applied to a column of Florisil®,
5) the Paclitaxel fraction from the column is purified by double crystallization,
6) the so obtained crystalline Paclitaxel is subjected to chromatography on a silica column. The Paclitaxel is separated from the other taxanes (related analogs, cephalomannine, etc.) in this step,
7) the purified Paclitaxel obtained from the previous step is crystallized twice, and
8) unseparated mixtures and mother liquors are recycled through the silica column to obtain additional amounts of pure Paclitaxel.

Of course, there are other processes for purifying Paclitaxel from natural sources, as is explained in the following references:
Kingston et al., who disclose new taxanes obtained from *Taxus brevifolia (J.* Nat. Prod. 1982; 45: 466-470); and
Witherup et al., who disclose a method for the separation of Paclitaxel and related compounds from *Taxus brevifolia (J.* Liq. Chrom. 1989; 12: 2117-2132).

US patent No. 5,279,949 issued to Nair in 1994 discloses the use of tissue from ornamental yew (*Taxus x media Hicksii*) for Paclitaxel purification. In this patent:
1) fresh needles are extracted with 70% alcohol,
2) the extract is decolorized with charcoal and filtered,
3) the filtered extract is concentrated to remove most of the organic solvent,
4) the aqueous concentrate is centrifuged to separate the solids which contain Paclitaxel,
5) the solids are then subjected to a first normal phase silica chromatography,
6) the obtained crude Paclitaxel fraction is subjected to a second, low pressure silica chromatography; and
7) final purification is carried out in a reverse phase column.

US patent No. 5,654,448 issued to Pandey et al., discloses a process for extraction and purification of Paclitaxel from barks of *Taxus brevifolia*. In this process:
1) the barks are treated thrice with methanol, each extraction being performed over a period of 5 days, the so-obtained extract being then concentrated;
2) the methanol concentrate is separated to obtain methylene chloride and water and the methylene chloride extract is evaporated to dryness, the solid residue containing the Paclitaxel amounting to about 1.8-2.2% w/w;
3) the solid residue is dissolved in acetone and mixed with one volume of hexane to remove polar impurities, and the mixture is evaporated to 1/3 of its volume;
4) the acetone/hexane residue is added drop-wise to hexane (1.5 L-3.0 L of residue for 10-15 L of hexane) to yield a precipitate which is filtered and dried under high vacuum (1mm to 2mm) at a temperature of 40°C to yield about 0.5-0.6 kg of a solid residue;
5) the solid residue obtained in the previous step is dissolved in 0,5 L of acetone-methylene chloride to form a 1:9 v/v mixture and subjected chromatography in a silica column; Cephalomannine is co-eluted out with Paclitaxel; the fractions containing Paclitaxel and Cephalomannine are pooled together and rotary evaporated to dryness, the solid residue being a crude mixture of Paclitaxel and Cephalomannine containing from 36 to 40 grams of Paclitaxel (45-55%);
6) a crude mixture (10g) obtained in the previous step is chemically modified to separate Paclitaxel from Cephalomannine by bromination; the solid residue obtained after bromination reaction having a weight of 13.2 g;
7) the brominated residue is dissolved in acetone/methylene chloride (1:9 v/v) and chromatographically separated over a silica column, the fractions containing Paclitaxel are pooled together and evaporated to dryness; and
8) the solid residue obtained in the previous step is dissolved in acetone and crystallized with an equal volume of n-hexane or other hexanes; and the crystals are washed with cold acetone/hexane, 1/1 v/v solution, filtered, and dried under vacuum at 40°C, the so obtained solid crystals weighing 4.84 g and containing > 97% w/w of Paclitaxel as measured by HPLC chromatography.

US patent Nos. 5,475,120 and 5,670,673 issued to Rao discloses a process for the isolation of Paclitaxel. In this process:
1) barks, needles, wood, roots, or a combination thereof, are treated with an alcohol and the obtained extract is concentrated under pressure (<35-40°C) to remove most of the alcohol;
2) the concentrate is partitioned with chloroform (or dichloromethane, dichloroethane or trichloroethane), the chloroform extract is concentrated under reduced pressure to form a thick syrup, and the syrup is poured into glass dishes and dried in a vacuum oven (<40°C) to form a powder if barks or wood are used, or needle-shaped crystals if needles are used (from 100 kg of the wood or barks, the yield of extract was 1.5-2.5 kg and from 100 kg of needles, it was 2.4-4.8 kg);
3) chloroform solid extracts (2-2.5 kg) are dissolved in acetonitrile (5 L) and water (5 L) was added; the mixture was then equilibrated with silica gel (2-3 L of slurry); more water (15 L) is added gradually while stirring the mixture; the clear solution is siphoned off and the thick slurry of silica gel which is now impregnated with the sample, is transferred onto the top of a stainless steel column; fractions containing Paclitaxel and various important Paclitaxel analogs, are set aside in a hood where slow evaporation of the solvent is carried out; crystals forming after in 1 to 2 days and the process is allowed to continue for 8-10 days; and
4) the so-obtained crude crystals which have less than 5% of Cephalomannine, are crystallized twice with a acetone/ligroin mixture and by using charcoal, thereby yielding 41 g of pure Paclitaxel, *viz.* a yield of 0.04%, the above yield being obtained with 100 kg of bark; alternatively, the crude crystals are decolorized by passing a solution in chloroform through a short column of silica or Florisil®. Another alternative method for removing the Cephalomannine contaminant consists of using ozone. Washing of the column with a mixture of 1-2% methanol in chloroform gave a bulk of Paclitaxel which can be recovered by concentration and crystallization. The yield is nearly the same as before, 40 g. HPLC chromatography analysis shows that the so recovered Paclitaxel contains less than 0.3% of Cephalomannine.

US patent no. 5,969,165 issued to Liu discloses a process for the isolation and purification of Paclitaxel and other related compounds from *Taxus canadensis by* industrial preparative low pressure chromatography.

In this process, needles and twigs of *Taxus canadensis* (200 kg) are extracted with 1000 L of methanol at 60°C for 5 hours and then filtered. The raw materials are extracted with 700 L of methanol at 55-60°C for another 4 hours and filtered. The filtrate is combined and mixed with 10 kg of activated carbon (5% w/w). The activated carbon is removed by filtration. The filtrate is then concentrated to approximately 100 L. Then 300 L of water and dichloromethane (1:1) are added. The organic layer is collected and the aqueous solution is extracted two more times with 200 L of dichloromethane. The dichloromethane solution is combined and evaporated to form a slurry and then diluted with 20 L of acetone.

The acetone solution is coated onto 20 kg of Celite® 545 and dried and then loaded onto the top of three industrial low pressure chromatographic columns (150 times.15 cm). Each column is packed with 15 kg of an aluminium oxide absorbent. The columns are eluted with a solvent system consisting of a mixture of hexane and acetone under a pressure between 10 and 15 psi with a flow rate of about 150 ml/min.

Fractions containing taxanes are collected and combined and then are concentrated to remove all other solvents. The resulting material is dissolved in methanol and kept at room temperature overnight to yield needle-like crystals. The crystals are filtered and recrystallized from acetone to yield white needle-like crystals identified as 13-acetyl-9-dihydrobaccatin III.

The filtrate is concentrated to dryness. The residue is dissolved in 3 L of acetone. The acetone solution is mixed with 1.5 kg of a polystyrene-divinylbenzene. The solvent is removed by evaporation and the resulting powder is loaded on top of an industrial low-pressure chromatographic column packed with polystyrene-divinylbenzene, and is eluted with 45% acetone in water at a flow rate of 150 ml/min under an operating pressure below 30 psi.

Fractions containing Paclitaxel and Cephalomannine are combined and evaporated to remove most of the acetone, then diluted with deionized water and extracted three times with 2,5 L of dichloromethane. The organic layer is concentrated to dryness and the residue is dissolved in 1 L of methanol.

Approximately 30% (v/v) of water is added to the methanol solution and the mixture is warmed to 60°C for a few minutes, and then kept at room temperature overnight. The crude crystalline solid from the methanol solution is filtered out and dried in a vacuum oven at 70-75°C. The solid consists of approximately 70% Paclitaxel and 25% Cephalomannine in a yield of 31 grams for 200 kg of needles and twigs of *Taxus canadensis.*

The crude Paclitaxel is dissolved in 200 ml of acetonitrile and diluted with 250 ml of deionised water and loaded to a chromatographic column. The chromatographic column is packed with a polymer resin (Diaion®, which is a macroporous polymethacrylate resin). The column is eluted with a step gradient of 35, 40, 45 and 50% acetonitrile in water.

The obtained fractions containing Paclitaxel or Cephalomannine are separately combined and kept at 5°C until crystallization. Then, Paclitaxel and Cephalomannine crystals are filtered separately and both are recrystallized at 65°C with a mixture of methanol and water.

Paclitaxel is obtained as white needle-like crystals with a purity > 99% at yield of 18.5 g (0.009%). Cephalomannine is obtained with a purity > 98% at a yield of 6.5 g (0.003%).

Thus, review of the known processes for the purification of Paclitaxel as disclosed in the prior art known to the Applicant shows that to obtain a high purity Paclitaxel, one must carry out numerous steps of separation by chromatography and purification by crystallization. This leads to a very high production cost due in particular to the low of Paclitaxel in the different species of Taxus. Moreover, the amount of biomass which can be purified, is very limited because of the small sizes of the chromatographic columns and because of the low yield in Paclitaxel obtained after purification.

### OBJECTS AND SUMMARY OF THE INVENTION

A first object of the present invention is to provide a process which permits:
- to make easier the obtention of a biomass after extraction of the barks, needles and/or branches of Taxus of different species;
- to increase the amount of biomass which Is so obtained and has to be purified by chromatography;
- to reduce the steps of purification;
- to increase the amount of obtained Paclitaxel; and finally
- to reduce the production cost to a more economical level.

Another object of the Invention is to provide a mixture of Paclitaxel crystals having a high purity.

In accordance with the present invention, the first object mentioned hereinabove is achieved with process for the extraction and purification of Paclitaxel from a natural source of taxanes containing the Paclitaxel to be extracted, which process comprises the following steps:
a) extracting, with an organic solvent, a raw material comprising Paclitaxel from said natural source of taxanes;
b) treating said raw material in a basic medium or an acidic medium in order to obtain a biomass by precipitation, isolating said biomass and drying it;
c) percolorizing the so-isolated biomass by removing resin and natural pigments contained therein, dissolving said biomass in acetone and then adding thereto at least one non-polar solvent until a Paclitaxel-enriched oily phase is obtained;
d) treating the biomass contained in the Paclitaxel-enriched oily phase recovered in the preceding step with an acidic medium when the treatment of step (b) has been carried out with a basic medium, or with a basic medium when the treatment of step (b) has been carried out with an acidic medium In order to obtain another biomass by precipitation, isolating said other biomass and drying it;
e) chromatographically purifying at least once a solution of the isolated other biomass obtained in the preceding step in a volatile solvent, and crystallizing at least once the purified solution obtained by chromatography.

In accordance with the invention, the second object mentioned hereinabove is achieved by a mixture of Paclitaxel crystals, which is obtained by the above process and which, after filtration and drying of the crystals consists of:
- about 53% of crystals having a purity higher than 99%,
- about 22% of crystals having a purity higher than 98%, and
- about 23% of crystals having a purity higher than 92%.

The present invention, its advantages and the way it can be reduced to practice will be better understood upon reading the following non-restrictive description.

### BRIEF DESCRIPTION OF THE DRAWINGS

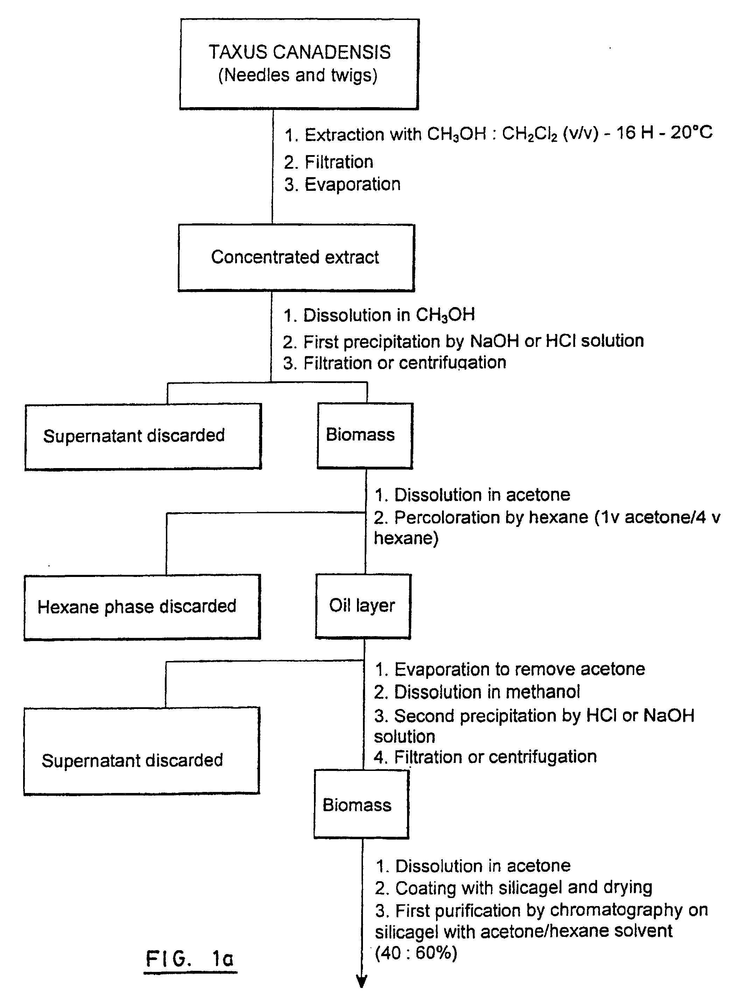

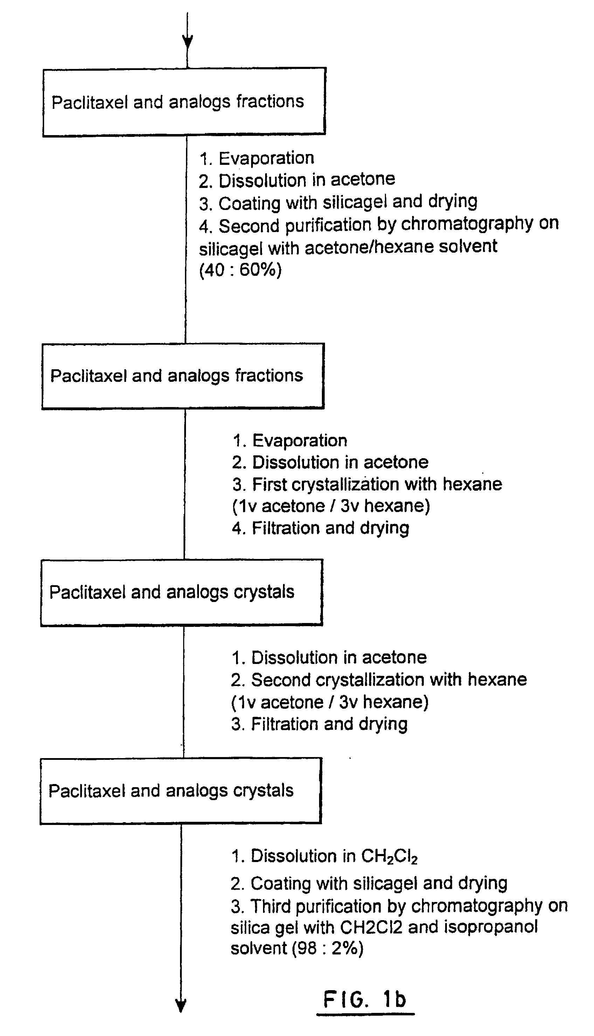

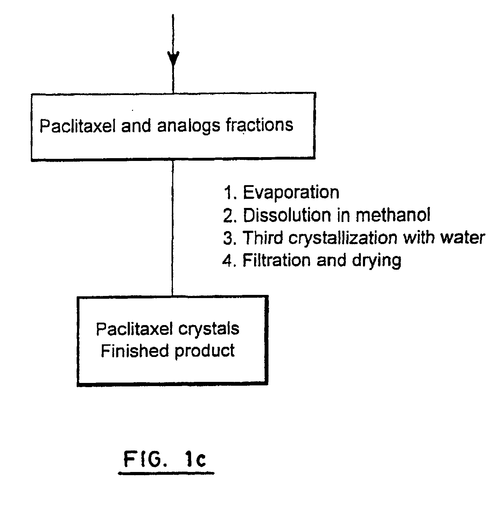

Figures 1a, 1b and 1c are a flow chart illustrating the process according to the invention.

### DETAILED DESCRIPTION OF THE INVENTION

Within the scope of the present invention and in the following description, the expression "to percolorize" means "to remove resin and pigments from a solution by adding a non polar solvent which may be miscible or unmiscible with the solution".

As aforesaid, the process according to the invention is intended to be used for the extraction and purification of Paclitaxel from a natural source of taxanes containing the Paclitaxel to be extracted.

### Step 1 - Extraction

The first step of the process consists of extracting a raw material containing Paclitaxel and its analogs from a natural source of taxanes, using an organic solvent mixture to do so.

The natural source of taxanes used as starting material for carrying out the process according to the invention is of *Taxus* type. More particularly, it consists of any one of the species of conifers that contain Paclitaxel. Such species of conifer containing Paclitaxel may consist of *Taxus brevifolia, Taxus baccata, Taxus canadensis, Taxus wallichiana, Taxus yunnanensis, Taxus densiformis, Taxus hicksii, Taxus wardii, Taxus cuspidata, Taxus capitata or Taxus brownii.*

The process according to the invention has the advantage of being usable with any part of the natural source of taxanes which contains Paclitaxel. Preferably, the extraction is carried out from barks of the selected conifer(s). Alternatively, the extraction can be carried out by simultaneously using branches and needles of the selected conifers.

Preferably, the extract that is so obtained is filtered to remove deposits, and is decanted in a double wall tank in which hot water (preferably at a temperature of 65-70°C) is fed. The solvent is distilled from this tank until a very thick liquid is obtained. The extract is then recovered and dissolved in methanol.

In the extraction step, the organic solvent that is used, is preferably selected from the group consisting of alcohols, halogenated hydrocarbons and mixtures of halogenated alcohols and hydrocarbons. As examples of such solvents, reference can be made to methanol, dichloromethane, trichloromethane and mixtures of methanol with dichloromethane or trichloromethane.

In the case where one uses a mixture of halogenated hydrocarbon and alcohol, the alcohol and halogenated hydrocarbon are present in volume ratio preferably comprised between 9:1 and 1:9. More preferably, the volume ratio of the mixture is equal to about 1:1.

### 2 - First precipitation

The second step of the process consists of treating the raw material either in a basic medium or an acidic medium to obtain precipitation of a biomass.

In the case where the precipitation is carried out in a basic medium, this medium preferably consists of a solution of a basic salt such as sodium acetate, potassium acetate or tris(hydroximethyl)aminomethane (also called TRIS).

According to a preferred embodiment, the basic medium consists of a solution of sodium hydroxide and the sodium hydroxide is used in a concentration ranging between 10 millimoles and 80 millimoles per liter of solution and more preferably between 20 millimoles and 50 millimoles per liter of solution.

The pH of the basic medium is usually comprised between 8.5 and 11, preferably between 9 and 10.

When the precipitation is carried out in an acidic medium, the acidic medium usually consists of a mineral acid solution or an organic acid solution. The acid can be selected from the group consisting of chlorhydric, acetic and citric acids.

The concentration of acid in the solution is preferably comprised between 10 millimoles and 50 millimoles per liter of solution, and more preferably between 20 millimoles and 30 millimoles per liter of solution.

The pH of the acidic medium is preferably comprised between 2 and 4.

In all cases, the volume of the basic or acidic solution added to the raw material to achieve precipitation of the biomass is equal to 1 to 20 times the volume of the raw material extracted in the first step of the process.

When use is made of a basic solution, the volume of basic solution added to the raw material is preferably equal to 1 to 15 times the volume of the raw material. When use is made of an acidic solution, the volume of acidic solution added to the raw material is preferably equal to 1 to 10 times the volume of raw material obtained in the first step of the process.

Under these conditions, the precipitate that is formed is very fine and light and its filtration is very difficult. As a matter of fact, the precipitate cannot be totally recovered by centrifugation at low speed (5,000 rpm). A speed of 15,000 or even 20,000 rpm is necessary to efficiently separate the precipitate from the solution.

To solve this problem and facilitate isolation of the precipitate from the so obtained biomass, the biomass may be salted before isolation and drying by addition of sodium chloride in a concentration comprised between 25 and 200 grams per liter of the acidic or basic solution used for the precipitation. Preferably, sodium chloride is added in a concentration comprised between 50 and 100, and more preferably between 50 and 75 grams per liter of acidic or basic solution used for the precipitation.

Salting the solution with sodium chloride permits to obtain a flocculation. The obtained precipitate is then heavier, more agglomerated and easier to filter or centrifuge at low speed.

Sodium chloride is preferably added quickly to the solution under heavy stirring.

According to another preferred embodiment of the invention, the biomass precipitated in this step is separated from the solution by filtration or by centrifugation and then dried at ambient air or under vacuum, preferably by ventilation or lyophilization.

### Step 3 - Percolorization

The third step of the process according to the invention consists of percolorizing the biomass isolated in the preceding step.

More specifically, in this third step, the biomass obtained from the preceding step of precipitation is put back in solution by adding thereto a volume of acetone equal to about 2 volumes of the raw material extracted in step 1, before precipitation.

More preferably, the biomass is put back in solution by first adding acetone and then by adding water. Water is preferably added at a ratio of 2 to 10 volumes, and more preferably 5 to 7 volumes, per 100 volumes of added acetone.

After such a dissolution, at least one non-polar solvent is added to the obtained solution to form a Paclitaxel-enriched oily phase.

The non-polar solvent(s) used in this percolorization step is (are) preferably selected from the group consisting of hydrocarbons miscible with acetone, such as hexane or heptane. When use is made of hexane, the volume of hexane that is used is usually 3 to 4 times that of the acetone solution.

The mixture that is obtained is then transferred into a decanting flask and the oily phase containing Paclitaxel and the other taxanes that are deposited at the bottom of the flask, are recovered. The oily phase is then evaporated and replaced by methanol.

This third step permits to eliminate most of the resin and natural pigments of the conifers used as starting material.

### Step 4 - Second precipitation

The fourth step of the process according to the invention consists of treating with a base or an acid the biomass contained in the Paclitaxel-enriched oily phase recovered in the preceding step to obtain another biomass by precipitation, used then isolating this other biomass and drying it.

More specifically, in this fourth step, the Paclitaxel-enriched phase obtained in the preceding step is first evaporated to dryness and put back in solution with methanol.

The so-obtained methanol solution is then precipitated in a basic medium when the first precipitation step hereinabove called step 2 has been carried in an acidic medium, or in a basic medium when the first precipitation step has been carried out in an acidic medium. The purpose of this precipitation is to obtain a biomass containing Paclitaxel in the form of a precipitate.

According to a preferred embodiment of the invention, the biomass that is so-obtained by precipitation, is further salted before isolation and drying, by adding thereto sodium chloride at a concentration comprised between 30 and 200 grams per liter of the acidic or basic solution used for the precipitation. Preferably, sodium chloride is added at a concentration comprised between 50 and 100 grams per liter of the acidic or basic solution used for the precipitation.

The precipitate contained in the biomass is then filtered and dried at ambient air or under vacuum, or is lyophilized.

The precipitate formed in this step is very fine and light, thereby making its filtration very difficult. As a matter of act, this precipitate can only be recovered partially if the centrifugation is carried out at a low speed (for example 5 000 rpm). Only a speed of 15 000 and 20 000 rpm would allow full separation of the precipitate from its solution. Thus, it is once again suitable to add sodium chloride to the solution to obtain a flocculation which makes the precipitate heavier, more agglomerated and easier to filter or centrifuge at low speed. The concentration of sodium chloride used for this treatment is preferably from 25 to 100 g per liter of solution, and more preferably from 50 to 75 g per liter of solution. Sodium chloride is added quickly into the solution under heavy stirring.

### Step 5 - Chromatographic purification

The fifth and last step of the process according to the invention consists of chromatographically purifying at least once a solution of the isolated other biomass obtained in the preceding step in a volatile solvent, and crystallizing at least once the purified solution obtained by chromatography.

To do so, the precipitate obtained after drying in step 4 is dissolved in a volatile solvent and then submitted to at least one step of chromatographic purification and at least one step of crystallization of the purified solution obtained by chromatography. Preferably however, the precipitate obtained after drying in step 4 is submitted to several chromatographic purifications and several crystallizations, the number of successive purifications and crystallizations being preferably equal to three.

These three successive purifications and crystallizations will now be described as sub-steps A to F.

### A - First chromatographic purification

In the first chromatographic purification step, the biomass obtained in step 4 of the process is dissolved in a volatile solvent. The obtained solution is mixed with silica gel and dried under ventilation, and then the silica gel covered with the biomass is loaded onto a chromatographic column containing the same type of gel. In this column, the biomass is purified with an elution mixture comprising from 30 to 40% acetone and from 60 to 70% hexane. Preferably, the elution mixture comprises about 40% acetone and about 60% hexane.

The volatile solvent used for the dissolution is preferably acetone. The acetone solution is then filtered to remove the insoluble particles and mixed with silica gel. The so obtained mixture is then dried under ventilation or under vacuum.

The gel impregnated with Paclitaxel and its analogs is loaded onto the column which preferably has a height of 142 cm and an inside diameter of 76 cm and which contains 2.2-2.3 kg silica gel. The gel of the column is washed and balanced with the elution mixture consisting of acetone and hexane (40-60%, volume per volume). Elution of the fractions is carried out with the same solvent mixture at a flow rate of about 100 ml/min, under a pressure varying from 0 to 30 psi.

### B- Second chromatographic purification

In the second chromatographic purification step, the Paclitaxel-enriched fractions recovered in the preceding step are preferably combined and then evaporated to dryness until a residue is obtained.

A solution of the residue is then prepared by solubilisation in a volatile solvent. The so obtained solution is then repurified by chromatography under the same conditions as in the preceding step to provide new Paclitaxel-enriched fractions.

According to a preferred embodiment, the residue is dissolved in acetone, and then mixed with silica gel and dried.

The gel impregnated with Paclitaxel and its analogs is loaded onto a column (142 cm long x 7.6 cm inside diameter) containing 2.2-2.3 kg silica gel. The gel of the column is washed and balanced with an elution mixture consisting of acetone and hexane (40-60%, volume per volume). Elution of the fractions is carried out with the same solvent mixture at a flow rate of about 100 ml/min, under a pressure of between 0-30 psi.

The fractions containing the Paclitaxel (in general from 40 to 60% Paclitaxel) are once again combined.

### C - First crystallization

In this step, the fractions containing Paclitaxel obtained by chromatography in the preceding purification step, are evaporated to dryness and put back In solution in acetone. The amount of acetone is adjusted to obtain an absorbency of solution having a value from 1.0 to 1.5 O.D. (optic density) for the peak corresponding to Paclitaxel according to HPLC analysis. Then, the Paclitaxel is crystallized by adding from 3 to 4 volumes of hexane in the acetone solution.

Crystals are formed rapidly. The mixture is left to rest overnight at ambient temperature or at a temperature comprised between 2 and 8°C to complete the crystallization.

### D - Second crystallization

In this step, the crystals obtained by recrystallization in the preceding step are separated by filtration and put back in solution in acetone with a volume of acetone adjusted to obtain an absorbency of the solution varying from 1.0 to 1.5 O.D. for the peak corresponding to Paclitaxel according to HPLC analysis.

The Paclitaxel contained in this solution is then recrystallized by adding to the acetone solution 3 to 4 volumes of hexane per volume of solution.

The crystals obtained in this step have, according to HPLC analysis, a Paclitaxel purity higher than 80%.

### E - Third chromatographic purification

In this step, the crystals obtained in the preceding step are filtered and then put back in solution with methylene chloride.

The so obtained solution Is then mixed with silica gel and dried under ventilation.

The silica gel coated with Paclitaxel is loaded onto a chromatographic column containing the same type of gel. The Paclitaxel is then repurified for the third time with an organic-solvent based elution mixture. Preferably, the elution mixture comprises from 95 to 98% methylene chloride and from 2 to 5% isopropanol.

Preferably, in this step, the crystals are dissolved with methylene chloride, mixed with silica gel and dried. The gel impregnated with Paclitaxel is loaded onto a chromatographic column (76 cm long x 7.6 cm inside diameter) containing 1.5-1.6 kg of silica gel. The gel of the column is washed and balanced with a solvent consisting of methylene chloride and isopropanol (98-2% per volume). Elution of the fractions is carried out with the same mixture of solvents, at a flow rate is about 50 ml/min under the pressure ranging between 0-30 psi.

### F - Third crystallization

In this step, the enriched fractions containing Paclitaxel recovered by chromatography in the preceding step are combined according to their purity, preferably according to purities of +98 to +99% and 90 to 98%. Then, they are evaporated to dryness and put back in solution in an alcohol, preferably methanol, a cetone or an alcohol acetone mixture.

The volume of added solvent, preferably methanol, is adjusted to obtain an absorbency varying from 1.0 to 1.5 O.D. for the peak corresponding to Paclitaxel according to HPLC analysis.

The Paclitaxel is then recrystallized for a third time by adding to the methanol solution from 2 to 10 volumes of water per volume of solution. Preferably, use is made of 4 to 7 volumes of water per volume of methanol solution.

In the last step of purification and of recrystallization disclosed hereinabove, the water that is used is preferably is a purified water obtained by delonization and/or distillation.

According to a preferred embodiment, 7 to 10 volumes of pure water cooled to a temperature of 2 to 4°C are added to a volume of the methanol solution cooled In ice. Then, the crystals appear immediately. Crystallization is continued all night at a temperature comprised between 2-4°C.

Addition of water in the methanol solution can be done at ambient temperature. The formation of the crystals will then be slower but it will be completed overnight at 2-4°C. The crystals are filtered and dried under ventilation or under vacuum, in order to obtain a fine and detached powder. The crystals are then put back in suspension in pure water and lyophilized during 66 to 72 hours at a temperature of about -60°C.

The obtained fractions of taxanes can be analysed by HPLC chromatography (Waters system) using an Autosampler (Waters 717 plus), a Photodiode Array Detector (Waters 996), a Multisolvent Delivery System (Waters® 600E) and a C18 Nova-Pak® column, 60 Å, 4µm (3.9 x 150 mm).

Analysis of the fractions is carried out by injecting a volume of 5 µl. The flow rate of the mobile phase is about 1ml/min, when using a solvent gradient: acetonitrile-water-methanol (20: 50: 30 at the beginning; 35: 35: 30 at the end).

The peaks of the compounds are detected at 228 nm and the time of analysis of a sample is about 36 minutes.

Preferably, the volatile solvent used in the chromatographic purification step for the solubilization of the residue is selected from the group consisting of cetones, C1-C3 light alcohols, ethyl acetate, methylene chloride and mixtures of these solvents.

At the end of step F, after crystallization of the Paclitaxel in water and after filtering and drying, a mixture of Paclitaxel crystals is obtained. This mixture of Paclitaxel crystals consists of:
- 53% of crystals having a purity higher than 99%;
- 22% of crystals having a purity higher than 98%; and
- 23% of crystals having a purity higher than 92%.

The following examples are given for the sole purpose of illustration and should not be considered as to limit the scope of the present invention.

### EXAMPLE 1

### Extraction

50 kg of dried needles and twigs of *Taxus canadensis* were washed with deionised water and dried under ventilation. The raw materials were ground and transferred into a cotton bag. This bag was put into a stainless steel tank containing 150 L of a solvent mixture (methylene chloride - methanol; 1:1 volume per volume). The extraction was carried out during 16 hours at ambient temperature. The extract was pumped through filters into a second double wall tank. The solvent was distilled with the aid of hot water at 70°C circulating in the double wall of the tank until a very thick liquid was obtained.

### First precipitation

The extract concentrate was recovered and diluted with 2 L of methanol. The biomass was isolated by precipitation by adding the extract to a NaOH solution at 20 mM. A very fine precipitate was formed as the extract was added to the NaOH solution. The volume ratio of the extract to the basic solution was about 1:15.

This fine precipitate was very difficult to filter. Only a centrifugation at very high speed (15 000-20 000 rpm) could allow recovery of the biomass. Thus, NaCl was added to the solution at a ratio of 50g/L of solution in order to make the precipitate more agglomerated. The precipitate was then filtered or centrifuged at a speed of only 4200 rpm at 20°C during 30 minutes (J6MC Beckman® Centrifugal machine, 4.2 JS rotor). It may be understood that a continuous flow centrifugal machine can be used to process large amounts of precipitate.

The so obtained precipitate was air or vacuum dried or was lyophilized (Freeze dryer - FTS Systems). The weight of the precipitate was about 500-550 g. This precipitate was solubilised in 2 L of acetone and filtered or centrifuged at 4200 rpm, at 0-2°C during 30 minutes, in order to remove the insoluble particles contained therein.

### Percolorization

Then, 1 L of the acetone solution was fed into a flask of 6 L, the solution was mixed with 4 L of hexane by successive addition of 1 L of hexane each followed by a heavy manual shaking. The mixture was allowed to rest for about 10 minutes, and the oily phase was recovered at the bottom of the flask.

In practice, the acetone solution can be treated in one batch by using a large Becher or an adequate tank from which the hexane phase can be siphoned off and the remaining liquid be transferred into a separating flask to complete recovery of the oily phase.

During this step, the highly coloured hexane phase was discarded.

### Second precipitation

The oily phase was then evaporated to dryness. The residue was dissolved in 2 L of methanol. The biomass was isolated for the second time by precipitation achieved by adding the methanol solution in a solution of HCI at 20 mM. A very fine precipitate was formed as the extract was added. The volume ratio of the extract to the acidic solution was about 1:5. Once again, this fine precipitate was very difficult to filter. Only a centrifugation at high speed (15 000-20 000 rpm) could allow recovery of the biomass. Thus, NaCI was again added to the solution (50 g/L of solution) to make the precipitate more agglomerated. The precipitate was then filtered or centrifuged at a speed of 4200 rpm only, at 20°C during 30 minutes (J6MC Beckman® Centrifugal machine, 4.2 JS rotor). Once again, a continuous flow centrifugal machine can be used to process large amounts of precipitate.

### First purification by chromatography on silica gel at low pressure

The recovered precipitate was dried with air or under vacuum or lyophilized. The weight of the precipitate was about 240-260 g. This precipitate was solubilised in 0.5 L of acetone and centrifuged at 4200 rpm, at 0-2°C, during 30 minutes to remove the insoluble particles contained therein. The acetone solution was then mixed with 200-250 g of silica gel (230-400 mesh, pharmaceutical quality, Silicycle®, Québec, Canada). The gel impregnated with the extract was air dried under ventilation or under vacuum. The dried gel was loaded onto a column (142 x 7.6 cm inside diameter) containing 2.2 kg of silica gel (230-400 mesh). The gel was washed and balanced with a mixture of acetone and hexane (40:60%, volume per volume). The elution was carried out with the same solvent, using a Dynamax® solvent delivery system. The flow rate of the elution was about 100 ml/min under a pressure between 10-30 psi. The volume of solvent mixture was about 30 L and each fraction was collected in batches using with 1 L of solvent purification. HPLC analysis indicated that from the 17^{th} to 25^{th} fractions obtained, there were 9 fractions which contained Paclitaxel.

The Paclitaxel contents in these fractions as determined by HPLC were respectively as follows: 11; 24; 32; 40; 38; 33; 29; 14 and 9%.

It is worth noting that the fractions containing Paclitaxel can be offset with respect to the others from one purification to the other.

### Second purification by chromatography on silica gel at low pressure

The fractions containing Paclitaxel were combined together and evaporated to dryness. The residue was resolubilised in 100 ml of acetone and put into contact with 100 g of silica gel. The gel coated with Paclitaxel was air dried under ventilation or under vacuum. The dried gel was loaded into a column (142 x 7.6 cm inside diameter) containing 2.2 kg of silica gel (230-400 mesh). The gel was washed and balanced with a mixture of acetone and hexane (40:60%, volume per volume). The elution was carried out with the same solvent using a solvent delivery system. The flow rate of the elution was about 100 ml/min under a pressure between 10-30 psi. The volume of solvent mixture was about 30 L and the fractions were collected in batches of using 1 L of solvent per fraction. HPLC analysis indicated that there were 5 fractions which contained Paclitaxel, from the 22^{nd} fraction to the 26^{th} fractions. The Paclitaxel content determined by HPLC In these fractions were as follows: 40; 58; 66; 50 and 49%. Once again, these fractions containing Paclitaxel can be offset with respect to the others from one purification step to the other.

### First crystallization

The fractions containing Paclitaxel were combined and evaporated to dryness. The residue was resolubilised in 800 ml of acetone. Adjustment of acetone volume was necessary on HPLC analysis so as to obtain an absorbency from 1.0 to 1.5 O.D. for the Paclitaxel peaks. 3 volumes of hexane (2.4 L) were added to the acetone solution. Crystals were formed in the following hour. The mixture was kept at a temperature comprised between 2-8°C overnight to complete the crystallization.

### Second crystallization

The obtained crystals were filtered and redissolved in 400 ml of acetone. 3 volumes of hexane (1.2 L) were added to the acetone solution. Crystals are formed In the following hour. The mixture was kept at a temperature comprised between 2-8°C overnight to complete the crystallization. The crystals were dried with air or under vacuum.

The dried weight of obtained crystals was about 10 g ± 0.2 g. HPLC analysis indicated that the Paclitaxel content was of about 80% and higher.

### Third purification by chromatography on silica gel at low pressure

The crystals were once again dissolved in 75 ml of methylene chloride and put into contact with 75 g of silica gel. The gel coated with Paclitaxel was air dried under ventilation or under vacuum. The dried gel was loaded onto a column (76 x 7.6 cm inside diameter) containing 1.5 kg of silica gel (230-400 mesh). The gel was washed and balanced with a mixture of methylene chloride and isopropanol (98:2%, volume per volume). The elution was carried out with the same solvent using a Dynamax® solvent delivery system. The flow rate of the elution was about 50 ml/min under a pressure between 10-30 psi. The volume of solvent mixture was about 30 L and the fractions were collected with 1 L of solvent per fraction. HPLC analysis indicates that there are 12 fractions which contain Paclitaxel, from the 11^{th} fraction to the 22^{nd} fractions. The Paclitaxel contents determined by HPLC in these fractions, were as follows: 80; 97; 99.50; 99.70; 99.70; 99.30; 98.60; 97.30; 94; 92; 89 and 85%. Once again, the fractions containing Paclitaxel can be offset with respect to the others from one purification step to the other.

### Third crystallization

The fractions containing Paclitaxel were combined to their respective purities and evaporated to dryness. The 12 ^{th} to 16^{th} fractions were dissolved in 100 ml of methanol (HPLC grade), 17^{th} to 18^{th} fractions were dissolved in 45 ml of methanol (HPLC grade) and the 19^{th} to 22^{th} fractions were dissolved in 75 ml of methanol (HPLC grade).
1) 700 ml of water (HPLC grade) cooled beforehand at 2-8°C were added to the methanol solution (12^{th} to 16^{th} fractions) cooled in ice. White crystals appeared immediately.
2) 300 ml of water (HPLC grade) cooled beforehand at 2-8°C were added to the methanol solution (17 ^{th} and 18 ^{th} fractions) cooled in ice. White crystals appeared immediately.
3) 500 ml of water (HPLC grade) cooled beforehand at 2-8°C were added to the methanol solution (19 ^{th} to 22 ^{th} fractions) cooled in ice. White crystals appeared immediately.

The crystals were separately filtered and dried with air or under vacuum. The dried weights and the purities of the crystals analysed by HPLC were as follows:
12^{th} to 16^{th} fractions: 3.40 g - Purity: 99.30%
17^{th} to 18^{th} fractions: 1.40 g - Purity: 98.85%
19^{th} to 22^{th} fractions: 1.60 g - Purity: 92.70%

Crystals which had a purity inferior to 98% were set aside and repurified together by chromatography under the same conditions as in the 3^{rd} purification step described above. This repurification allowed to obtain about 75% of the total amount of crystals with a purity higher than to 99%.

### EXAMPLE 2

The process disclosed in example 2 was similar to the one disclosed in example 1 except for the first and second precipitation steps that were carried as follows.

### First precipitation

The extract concentrate was recovered and diluted with 2 L of methanol. The biomass was isolated by precipitation by adding the extract to a solution of HCl at 20 mM. A very fine precipitate was formed as the extract was added. The volume of ratio of the extract with respect to the acidic solution was about 1:10. The fine precipitate was very difficult to filter. Only a centrifugation at high speed: (15 000-20 000 rpm) could allow recovery of the biomass. Thus, NaCI was added to the solution at a ratio of 50 g/L of solution in order to make the precipitate more agglomerated. The precipitate was centrifuged at 4200 rpm at 20°C during 30 minutes (J6MC Beckman® Centrifugal machine, 4.2 JS rotor).

The recovered precipitate was dried with air or under vacuum or lyophilized. The weight of the precipitate was about 500-520 g. This precipitate was solubilised in 2 L of acetone and filtered or centrifuged at 4200 rpm at 0-2°C during 30 minutes, to remove the insoluble particles contained therein.

### Second precipitation

The oily phase was then evaporated to dryness. The residue was dissolved in 2 L of methanol. The biomass was isolated for a second time by precipitation by adding the methanol solution to a solution of NaOH at 20 mM. A very fine precipitate was formed as the extract was added. The volume ratio of the extract with respect to the basic solution was about 1:5. This fine precipitate was very difficult to filter. Only a centrifugation at high speed (15 000-20 000 rpm) would have permit to recuperate the biomass. Therefore, NaCI was added to the solution in a ratio of 50 g/L of solution, in order to make the precipitate more agglomerated. The precipitate was then filtered and centrifuged at 4200 rpm at 20°C during 30 minutes (J6MC Beckman®, 4.2 JS rotor).

The yield and the purities of the Paclitaxel obtained in this example 2 were identical to those obtained in example 1.

The above examples show that the process according to the invention provides a substantial decrease in the production cost of Paclitaxel as compared to the different processes known in the art. This cost decrease is caused by an increase of the Paclitaxel yield with acceptable therapeutic purity and by noticeable savings in terms of non-recoverable products (solvents, gel...) and production time. In addition to these advantages, the present invention provides a very simple, fast and easy process that can be used at an industrial level without being limited by the low amount of Paclitaxel contained in the treated biomasses.

## Claims

1. Process for extraction and purification of Paclitaxel from a natural source of taxanes containing said Paclitaxel to be extracted, said process comprising the following steps:
a) extracting with an organic solvent, a raw material comprising Paclitaxel from said natural source of taxanes;
b) treating said raw material in a basic medium or an acidic medium in order to obtain a biomass by precipitation, isolating said biomass and drying it;
c) percolorizing the so-isolated biomass by removing resin and natural pigments contained therein, dissolving said biomass in acetone and then adding thereto at least one non-polar solvent until a Paclitaxel-enriched oily phase is obtained;
d) treating the biomass contained in the Paclitaxel-enriched oily phase recovered in the preceding step with an acidic medium when the treatment of step (b) has been carried out wiht a basic medium, or with a basic medium when the treatment of step (b) has been carried out with an acidid medium in order to obtain another biomass by precipitation, isolating said other biomass and drying it;
e) chromatographically purifying at least once a solution of the isolated other biomass obtained in the preceding step in a volatile solvent, and crystallizing at least once the purified solution obtained by chromatography.

2. Process according to claim 1, **characterized in that** the natural source of taxanes containing Paclitaxel consists of conifers selected from the group consisting of *Taxus brevifolia, Taxus baccata, Taxus canadensis, Taxus wallichiana, Taxus yunnanensis, Taxus densiformis, Taxus hicksii, Taxus wardii, Taxes cuspidata, Taxes capitata, Taxus brownii.*

3. Process according to claim 1 or 2, **characterized in that**, in the step (a), all parts of the conifers are processed.

4. Process according to any one of claims 1 to 2, **characterized in that**, in step (a), barks of the conifers are processed.

5. Process according to any one of claims 1 to 2, **characterized in that**, in step (a) branches and needles of the conifers are simultaneously processed.

6. Process according to any one of claims 1 to 5, **characterized in that**, in step (a), the organic solvent is selected from the group consisting of alcohols, halogenated hydrocarbons and mixtures of alcohols and halogenated hydrocarbons.

7. Process according to claim 6, **characterized in that**, in step (a), the solvent is a mixture of methanol and dichloromethane or a mixture of methanol and trichloromethane.

8. Process according to claim 6 or 7, **characterized in that** the alcohol and halogenated hydrocarbon are present in a volume ratio ranging from 9:1 to 1:9.

9. Process according to claim 8, **characterized in that** the volume ratio of alcohol to halogenated hydrocarbon is about 1:1.

10. Process according to any one of claims 1 to 9, **characterized in that**, in step (b), the biomass is precipitated in a basic medium consisting of a solution of a basic salt selected from the group consisting of sodium acetate, potassium acetate, sodium hydroxide and tris(hydroxymethyl)aminomethane.

11. Process according to claim 10, **characterized in that**:
- the basic salt is sodium hydroxide;
- the sodium hydroxide is used in a concentration comprised between 10 mM and 80 mM per liter of solution; and
- the pH of the solution is comprised between 8.5 and 11.

12. Process according to any one of claims 10 and 11, **characterized in that** the pH of the solution is comprised between 9 and 10.

13. Process according to any one of claims 1 to 10, **characterized in that** in step (b), the biomass is precipitated in an acidic medium consisting of a solution of a mineral or organic acid.

14. Process according to claim 13, **characterized in that** the acid is selected from the group consisting of hydrochloric, acetic and citric acids.

15. Process according to any one of claims 13 to 14, **characterized in that** the acid concentration of said solution is comprised between 10 mM and 50 mM per liter of solution.

16. Process according to claim 15, **characterized in that** the acid concentration is comprised between 20 mM and 30 mM per liter of solution.

17. Process according to any one of claims 13 to 16, **characterized in that** the pH of the solution is comprised between 2 and 4.

18. Process according to any one of claims 1 to 17, **characterized in that**, in step (b), the precipitation of the biomass is made with a solution of said base or acid in a volume ratio of said solution to the raw material extracted in step (a) comprised between 1 and 10.

19. Process according to any one of claims 1 to 18, **characterized in that** the precipitated biomass is salted before isolation and drying, by adding thereto sodium chloride in a concentration comprised between 25 and 200 g per liter of the solution used for the precipitation.

20. Process according to claim 19, **characterized in that** sodium chloride is added in a concentration comprised between 50 and 100 g per liter of the solution used for the precipitation.

21. Process according to any one of claims 1 to 20, **characterized in that** the precipitated biomass is separated by filtration or by centrifugation and then dried by ventilation or by lyophilization.

22. Process according to any one of claims 1 to 21, **characterized in that** said at least one non-polar solvent used in step (c) is selected from the group consisting of hexane or heptane.

23. Process according to any one of claims 1 to 22, **characterized in that** in step (c), the dried biomass obtained in step (b) is put back in solution by adding a volume of acetone equal to 2 volumes of the raw material extracted in step (a).

24. Process according to any one of claims 1 to 22, **characterized in that** the dried biomass is obtained in step (b) put back in solution by adding acetone and then adding water.

25. Process according to claim 24, **characterized in that** water is added at a ratio of 2 to 10 volumes per 100 volumes of added acetone.

26. Process according to any one of claims 1 to 25, **characterized in that**, in step (d):
- the Paclitaxel-enriched phase obtained in step (c) is evaporated to dryness and put back in solution in methanol; and
- said methanol solution is then precipitated in a basic medium when the first precipitation described in step (b) has been carried out in an acidic medium, or in an acidic medium, when the first precipitation described in step (b) has been carried out in a basic medium;
- so as to obtain a biomass containing Paclitaxel, said Paclitaxel being in the form of a precipitate.

27. Process according to any one of claims 1 to 26, **characterized in that** the biomass obtained in step (d) is further salted before isolation and drying by adding thereto sodium chloride at a concentration comprised between 30 and 200 g per liter of the solution used for the precipitation.

28. Process according to claim 27, **characterized in that** sodium chloride is added in a concentration comprised between 50 and 100 g per liter of the solution used for the precipitation.

29. Process according to any one of claims 1 to 28, **characterized in that** step (e) comprises the following sub-steps:
e₁) a first chromatographic purification comprising dissolving the precipitate isolated in step (d) in a volatile solvent, preparing a mixture of the so-obtained solution with silica gel, treating said mixture in a chromatographic column containing silica gel, and recovering Paclitaxel-enriched fractions;
e₂) a second chromatographic purification comprising by evaporating to dryness the Paclitaxel-enriched fractions recovered in the preceding step until a residue is obtained, and preparing a mixture by solubilizing said residue in a volatile solvent, said mixture being repurified by chromatography under the same conditions as in the preceding sub-step in order to obtain other Paclitaxel-enriched fractions;
e₃) a first crystallization comprising evaporating to dryness the other Paclitaxel-enriched fractions obtained in the preceding sub-step until a residue is obtained, preparing a mixture of said residue in acetone and crystallizing the Paclitaxel contained in the mixture with a non-polar solvent;
e₄) a second crystallization comprising solubilizing in acetone Paclitaxel crystals obtained in the preceding sub-step and recrystallizing the Paclitaxel under the same conditions as in the preceding sub-step;
e₅) a third chromatographic purification comprising solubilizing the crystals obtained by recrystallization in the preceding sub-step in a volatile solvent to obtain a solution, preparing a mixture of said solution with silica gel and treating said mixture in a chromatographic column containing silica gel in order to obtain, with an elution solvent, further Paclitaxel-enriched fractions; and
e₆) a third crystallization comprising evaporating to dryness the further Paclitaxel-enriched fractions obtained in the preceding sub-step until a residue is obtained, solubilizing the residue in an alcohol, acetone or an alcohol-acetone mixture to obtain another mixture, and crystallizing the Paclitaxel contained in the other mixture with water.

30. Process according to claim 29, **characterized in that**, in step (e₁):
- the biomass obtained in step (d) is put back in solution in a volatile solvent;
- the obtained solution is mixed with silica gel and dried under ventilation;
- the silica gel recovered from the biomass is loaded into a chromatographic column containing silica gel; and
- the biomass is then purified with an elution mixture containing from 30% to 40% acetone and from 60% to 70% hexane.

31. Process according to claim 30, **characterized in that** the elution mixture comprised about 40% acetone and about 60% hexane.

32. Process according to any one of claims 29 to 31, **characterized in that**, in step (e₂):
- the enriched fractions containing Paclitaxel obtained by chromatography in step (e₁), are regrouped, evaporated to dryness and put back in solution in a volatile solvent;
- the solution obtained is mixed with silica gel and dried under ventilation;
- the silica gel recovered from the biomass is loaded into a chromatographic column containing silica gel; and
- then the Paclitaxel is repurified by an organic solvent mixture comprising from 30% to 40% acetone and from 60% to 70% hexane.

33. Process according to claim 32, **characterized in that** the organic solvent mixture contains about 40% acetone and 60% hexane.

34. Process according to any one of claims 29 to 31, **characterized in that**, in step (e₃):
- the fractions containing Paclitaxel obtained by chromatography in step (e₂), are regrouped and evaporated to dryness and put back in solution in acetone, the quantity of acetone being adjusted so as to obtain an absorbency of said solution having a value of 1.0 to 1.5 O.D., for the peak corresponding to the Paclitaxel according to HPLC analysis; and
- the Paclitaxel is then crystallized by adding from 3 to 4 volumes of hexane in the acetone solution.

35. Process according to any one of claims 29 to 34, **characterized in that**, in step (e₄):
- the crystals obtained in step (e₃) are separated by filtration and put back in solution in acetone, the volume of acetone being adjusted so as to obtain an absorbency of said solution varying from 1.0 to 1.5 O.D. for the peak corresponding to the Paclitaxel according to HPLC analysis; and
- the Paclitaxel is then recrystallized by adding from 3 to 4 volumes of hexane in the acetone solution.

36. Process according to any one of claims 29 to 35, **characterized in that**, in step (e₅);
- the crystals obtained in step (e₄), are filtered and put back In solution in methylene chloride;
- the so obtained solution Is then mixed with silica gel and dried under ventilation;
- the silica gel covered with Paclitaxel is loaded onto a chromatographic column containing silica gel; and
- the Paclitaxel is then repurified for a third time with an organic-solvent based elution mixture.

37. Process according to claim 36, **characterized in that** the elution mixture comprises from 95% to 98% methylene chloride and from 2% to 5% isopropanol.

38. Process according to any one of claims 29 to 37, **characterized in that**, in step (e₆);
- the enriched fractions containing Paclitaxel obtained by chromatography in step (e₅), are regrouped according to their purity, evaporated to dryness and then put back in solution in methanol, the volume of methanol being added so as to obtain an absorbency varying from 1.0 to 1.5 O.D. for the peak corresponding to the Paclitaxel according to HPLC analysis; and
- the Paclitaxel is then recrystallized for a third time by adding from 2 to 10 volumes of water in the methanol solution.

39. Process according to claim 38, **characterized in that** the Paclitaxel is recrystallized for the third time by adding from 4 to 7 volumes of water in the methanol solution.

40. Process according to any one of claims 29 to 39, **characterized in that** the volatile solvent used in steps (e₁), (e₂) and (e₃) is selected from the group consisting of acetone, C1-C3 light alcohols, ethyl acetate, methylene chloride and mixtures of these solvents.

41. Process according to any one of claims 29 to 40, **characterized in that**, in step (e₆), after crystallization of the Paclitaxel in water, a mixture of Paclitaxel crystals is obtained by filtering and then dried.

42. Process according to any one of claims 1 to 41, **characterized in that** the water used in the whole process is purified by deionisation and/or by distillation.

## Patentansprüche

1. Verfahren zum Extrahieren und Reinigen von Paclitaxel aus einer natürlichen Quelle von Taxanen, die das zu extrahierende Paclitaxel enthält, umfassend die folgenden Schritte:
a) Extrahieren eines Paclitaxel enthaltenden Rohmaterials aus der natürlichen Taxan-Quelle mit einem organischen Lösemittel;
b) Behandeln des Rohmaterials in einem basischen oder einem sauren Medium, um durch Ausfällung eine Biomasse zu erhalten, Isolieren der Biomasse und Trocknen derselben;
c) Perkolorisieren der so isolierten Biomasse durch Entfernen von darin enthaltenem Harz und natürlicher Pigmente, Lösen der Biomasse in Aceton und dann Hinzugeben wenigstens eines unpolaren Lösemittels, bis eine mit Paclitaxel-angereicherte ölige Phase erhalten wird;
d) Behandeln der Biomasse, die in der mit Paclitaxel angereicherten, im vorhergehenden Schritt erhaltenen öligen Phase enthalten ist, mit einem sauren Medium, falls die Behandlung in Schritt b) mit einem basischen Medium erfolgte, oder mit einem basischen Medium, falls die Behandlung in Schritt (b) mit einem sauren Medium erfolgte, um durch Ausfällen eine andere Biomasse zu erhalten, Isolieren dieser anderen Biomasse und Trocknen derselben;
e) wenigstens einmaliges chromatographisches Reinigen einer Lösung der isolierten, im vorhergehenden Schritt gewonnenen anderen Biomasse in einem flüchtigen Lösemittel, und wenigstens einmaliges Kristallisieren der durch Chromatographie erhaltenen gereinigten Lösung.

2. Verfahren nach Anspruch 1, **dadurch gekennzeichnet, dass** die natürliche, Paclitaxel enthaltende Taxan-Quelle aus Koniferen besteht, die ausgewählt sind aus der Gruppe bestehend aus *Taxus brevifolia, Taxus baccata, Taxus canadensis, Taxus wallichiana, Taxus yunnanensis, Taxus densiformis, Taxus hicksii, Taxus wardii, Taxus cuspidata, Taxus capitata, Taxus brownii.*

3. Verfahren nach Anspruch 1 oder 2, **dadurch gekennzeichnet, dass** in Schritt (a) alle Teile der Koniferen verarbeitet werden.

4. Verfahren nach einem der Ansprüche 1 bis 2, **dadurch gekennzeichnet, dass** in Schritt (a) die Rinden der Koniferen verarbeitet werden.

5. Verfahren nach einem der Ansprüche 1 bis 2, **dadurch gekennzeichnet, dass** in Schritt (a) Zweige und Nadeln der Koniferen gleichzeitig verarbeitet werden.

6. Verfahren nach einem der Ansprüche 1 bis 5, **dadurch gekennzeichnet, dass** in Schritt (a) das organische Lösemittel ausgewählt ist aus der Gruppe bestehend aus Alkoholen, halogenierten Kohlenwasserstoffen und Mischungen aus Alkoholen und halogenierten Kohlenwasserstoffen.

7. Verfahren nach Anspruch 6, **dadurch gekennzeichnet, dass** in Schritt (a) das Lösemittel eine Mischung aus Methanol und Dichlormethan oder eine Mischung aus Methanol und Trichlormethan ist.

8. Verfahren nach Anspruch 6 oder 7, **dadurch gekennzeichnet, dass** der Alkohol und der halogenierte Kohlenwasserstoff in einem Volumenverhältnis im Bereich von 9:1 bis 1:9 vorliegen.

9. Verfahren nach Anspruch 8, **dadurch gekennzeichnet, dass** das Volumenverhältnis von Alkohol zu halogeniertem Kohlenwasserstoff etwa 1:1 beträgt.

10. Verfahren nach einem der Ansprüche 1 bis 9, **dadurch gekennzeichnet, dass** in Schritt (b) die Biomasse in einem basischen Medium ausgefällt wird, das aus einer Lösung eines basischen Salzes besteht, welches aus der Gruppe bestehend aus Natriumacetat, Kaliumacetat, Natriumhydroxid und Tris(hydroxymethyl)aminomethan ausgewählt ist.

11. Verfahren nach Anspruch 10, **dadurch gekennzeichnet, dass**:
- das basische Salz Natriumhydroxid ist;
- das Natriumhydroxid in einer Konzentraton zwischen 10 mM und 80 mM pro Liter der Lösung eingesetzt wird; und
- der pH-Wert der Lösung zwischen 8,5 und 11 liegt.

12. Verfahren nach einem der Ansprüche 10 und 11, **dadurch gekennzeichnet, dass** der pH-Wert der Lösung zwischen 9 und 10 liegt.

13. Verfahren nach einem der Ansprüche 1 bis 10, **dadurch gekennzeichnet, dass** in Schritt (b) die Biomasse in einem sauren Medium ausgefällt wird, welches aus einer Lösung eines Minerals oder einer organischen Säure besteht.

14. Verfahren nach Anspruch 13, **dadurch gekennzeichnet, dass** die Säure aus der Gruppe bestehend aus Salzsäure, Essigsäure und Zitronensäure ausgewählt ist.

15. Verfahren nach einem der Ansprüche 13 bis 14, **dadurch gekennzeichnet, dass** die Säurekonzentration der Lösung zwischen 10 mM und 50 mM pro Liter der Lösung liegt.

16. Verfahren nach Anspruch 15, **dadurch gekennzeichnet, dass** die Säurekonzentration zwischen 20 mM und 30 mM pro Liter der Lösung liegt.

17. Verfahren nach einem der Ansprüche 13 bis 16, **dadurch gekennzeichnet, dass** der pH-Wert der Lösung zwischen 2 und 4 liegt.

18. Verfahren nach einem der Ansprüche 1 bis 17, **dadurch gekennzeichnet, dass** in Schritt (b), das Ausfällen der Biomasse mit einer Lösung der Base oder der Säure bei einem Volumenverhältnis der Lösung zu dem in Schritt (a) extrahierten Rohmaterial zwischen 1 und 10 geschieht.

19. Verfahren nach einem der Ansprüche 1 bis 18, **dadurch gekennzeichnet, dass** die ausgefällte Biomasse vor dem Isolieren und Trocknen gesalzen wird, und zwar durch Hinzugeben von Natriumchlorid in einer Konzentration zwischen 25 und 200 g pro Liter der für die Ausfällung verwendeten Lösung.

20. Verfahren nach Anspruch 19, **dadurch gekennzeichnet, dass** Natriumchlorid in einer Konzentration zwischen 50 und 100 g pro Liter der für die Ausfällung verwendeten Lösung hinzugegeben wird.

21. Verfahren nach einem der Ansprüche 1 bis 20, **dadurch gekennzeichnet, dass** die ausgefällte Biomasse durch Filtration oder durch Zentrifugieren getrennt und dann durch Ventilation oder durch Lyophilisation getrocknet wird.

22. Verfahren nach einem der Ansprüche 1 bis 21, **dadurch gekennzeichnet, dass** das wenigstens eine unpolare Lösemittel, das in Schritt (c) verwendet wird, aus der Gruppe bestehend aus Hexan oder Heptan ausgewählt ist.

23. Verfahren nach einem der Ansprüche 1 bis 22, **dadurch gekennzeichnet, dass** in Schritt (c) die in Schritt (b) erhaltene, getrocknete Biomasse erneut gelöst wird, und zwar durch Zugabe eines Volumens von Aceton, das 2 Volumina des in Schritt (a) extrahierten Rohmaterials entspricht.

24. Verfahren nach einem der Ansprüche 1 bis 22, **dadurch gekennzeichnet, dass** die in Schritt (b) erhaltene, getrocknete Biomasse erneut gelöst wird, und zwar durch Zugabe von Aceton und anschließende Zugabe von Wasser.

25. Verfahren nach Anspruch 24, **dadurch gekennzeichnet, dass** Wasser in einem Verhältnis von 2 bis 10 Volumina pro 100 Volumina des hinzugefügten Acetons hinzugegeben wird.

26. Verfahren nach einem der Ansprüche 1 bis 25, **dadurch gekennzeichnet, dass** in Schritt (d):
- die in Schritt (c) erhaltene, mit Paclitaxel angereicherte Phase zur Trockne verdampft und erneut, in Methanol, gelöst wird; und
- die Methanol-Lösung dann in einem basischen Medium ausgefällt wird, falls die erste, in Schritt (b) beschriebene Ausfällung in einem sauren Medium durchgeführt wurde, oder in einem sauren Medium, falls die erste, in Schritt (b) beschriebene Ausfällung in einem basischen Medium durchgeführt wurde;
- so dass eine Paclitaxel enthaltende Biomasse erhalten wird, wobei Paclitaxel in Form eines Präzipitats vorliegt.

27. Verfahren nach einem der Ansprüche 1 bis 26, **dadurch gekennzeichnet, dass** die in Schritt (d) erhaltene Biomasse weiterhin vor dem Isolieren und Trocknen gesalzen wird, und zwar durch Hinzugeben von Natriumchlorid in einer Konzentration zwischen 30 und 200 g pro Liter der für die Ausfällung verwendeten Lösung.

28. Verfahren nach Anspruch 27, **dadurch gekennzeichnet, dass** Natriumchlorid in einer Konzentration zwischen 50 und 100 g pro Liter der für die Ausfällung verwendeten Lösung hinzugegeben wird.

29. Verfahren nach einem der Ansprüche 1 bis 28, **dadurch gekennzeichnet, dass** Schritt (e) die folgenden Unterschritte umfasst:
e₁) eine erste chromatographische Reinigung, umfassend: Lösen des in Schritt (d) isolierten Präzipitats in einem flüchtigen Lösemittel, Herstellen einer Mischung der so erhaltenen Lösung mit Silicagel, Behandeln der Mischung in einer Silicagel enthaltenden Chromatographiesäule und Gewinnung von mit Paclitaxel angereicherten Fraktionen;
e₂) eine zweite chromatographische Reinigung, umfassend: Verdampfen der in dem vorhergehenden Schritt gewonnenen, mit Paclitaxel angereicherten Fraktionen bis zur Trockne, bis ein Rückstand erhalten wird, und Herstellen einer Mischung durch Solubilisieren des Rückstandes in einem flüchtigen Lösemittel, wobei die Mischung erneut gereinigt wird, und zwar durch Chromatographie unter den gleichen Bedingungen wie in dem vorhergehenden Unterschritt, um andere, mit Paclitaxel angereicherte Fraktionen zu erhalten;
e₃) eine erste Kristallisation, umfassend: Verdampfen der anderen, in dem vorhergehenden Unterschritt gewonnenen, mit Paclitaxel angereicherten Fraktionen bis zur Trockne, bis ein Rückstand erhalten wird, Herstellen einer Mischung dieses Rückstands in Aceton und Kristallisieren des in der Mischung enthaltenen Paclitaxel mit einem unpolaren Lösemittel;
e₄) eine zweite Kristallisation, umfassend: Solubilisieren von im vorhergehenden Unterschritt erhaltenen Paclitaxel-Kristallen in Aceton und Rekristallisieren des Paclitaxel unter den gleichen Bedingungen wie in dem vorhergehenden Unterschritt;
e₅) eine dritte chromatographische Reinigung, umfassend: Solubilisieren der durch Rekristallisation in dem vorhergehenden Unterschritt erhaltenen Kristalle in einem flüchtigen Lösemittel, um eine Lösung zu erhalten, Herstellen einer Mischung der Lösung mit Silicagel und Behandeln der Mischung in einer Silicagel enthaltenden Chromatographie-Säule, um, mit einem Elutionslösemittel, weitere mit Paclitaxel angereicherte Fraktionen zu erhalten; und
e₆) eine dritte Kristallisation, umfassend: Verdampfen der in dem vorhergehenden Unterschritt erhaltenen, weiteren, mit Paclitaxel angereicherten Fraktionen zur Trockne, bis ein Rückstand erhalten wird, Solubilisieren des Rückstandes in einem Alkohol, in Aceton oder einer Alkohol-Aceton-Mischung, um eine andere Mischung zu erhalten, und Kristallisieren des in der anderen Mischung enthaltenen Paclitaxels mit Wasser.

30. Verfahren nach Anspruch 29, **dadurch gekennzeichnet, dass** in Schritt (e₁):
- die in Schritt (d) erhaltene Biomasse erneut, in einem flüchtigen Lösemittel, gelöst wird;
- die erhaltene Lösung mit Silicagel gemischt und unter Ventilation getrocknet wird;
- das aus der Biomasse rückgewonnene Silicagel auf eine Silicagel enthaltende Chromatographiesäule gegeben wird; und
- die Biomasse dann mit einer Elutionsmischung, die 30 % bis 40 % Aceton und 60 % bis 70 % Hexan enthält, gereinigt wird.

31. Verfahren nach Anspruch 30, **dadurch gekennzeichnet, dass** die Elutionsmischung etwa 40 % Aceton und etwa 60 % Hexan enthält.

32. Verfahren nach einem der Ansprüche 29 bis 31, **dadurch gekennzeichnet, dass** in Schritt (e₂):
- die Paclitaxel enthaltenden, angereicherten Fraktionen, welche durch Chromatographie in Schritt (e₁) erhalten wurden, neu gruppiert, zur Trockne verdampft und erneut, in einem flüchtigen Lösemittel, gelöst werden;
- die erhaltene Lösung mit Silicagel gemischt und unter Ventilation getrocknet wird;
- das aus der Biomasse rückgewonnene Silicagel auf eine Silicagel enthaltende Chromatographiesäule gegeben wird; und
- das Paclitaxel dann mit einer Mischung organischer Lösemittel, die 30 % bis 40 % Aceton und 60 % bis 70 % Hexan umfasst, erneut gereinigt wird.

33. Verfahren nach Anspruch 32, **dadurch gekennzeichnet, dass** die Mischung organischer Lösemittel etwa 40 % Aceton und 60 % Hexan enthält.

34. Verfahren nach einem der Ansprüche 29 bis 31, **dadurch gekennzeichnet, dass** in Schritt (e₃):
- die in Schritt (e₂) durch Chromatographie erhaltenen, Paclitaxel enthaltenden Fraktionen neu gruppiert und zur Trockne verdampft und erneut, in Aceton, gelöst werden, wobei die Menge des Acetons so eingestellt wird, dass ein Absorptionsvermögen der Lösung mit einem Wert von 1,0 bis 1,5 O.D. erhalten wird für den Paclitaxel entsprechenden Gipfel gemäß der HPLC-Analyse; und
- das Paclitaxel dann durch Zugabe von 3 bis 4 Volumina Hexan zu der Acetonlösung kristallisiert wird.

35. Verfahren nach einem der Ansprüche 29 bis 34, **dadurch gekennzeichnet, dass** in Schritt (e₄):
- die in Schritt (e₃) erhaltenen Kristalle durch Filtration getrennt und erneut, in Aceton, gelöst werden, wobei das Volumen des Acetons so eingestellt wird, dass ein Absorptionsvermögen der Lösung erhalten wird, das zwischen 1.0 bis 1.5 O.D. für den Paclitaxel entsprechenden Gipfel gemäß der HPLC-Analyse variiert.
- das Paclitaxel dann durch Zugabe von 3 bis 4 Volumina Hexan zu der Aceton-Lösung rekristallisiert wird.

36. Verfahren nach einem der Ansprüche 29 bis 35, **dadurch gekennzeichnet, dass** in Schritt (e₅):
- die in Schritt (e₄) erhaltenen Kristalle gefiltert und erneut, in Methylenchlorid, gelöst werden;
- die so erhaltene Lösung dann mit Silicagel gemischt und unter Ventilation getrocknet wird;
- das mit Silicagel bedeckte Paclitaxel auf eine Silicagel enthaltende Chromatographiesäule gegeben wird; und
- das Paclitaxel dann zum dritten Mal, mit einer auf organischem Lösemittel basierenden Elutionsmischung, gereinigt wird.

37. Verfahren nach Anspruch 36, **dadurch gekennzeichnet, dass** die Elutionsmischung 95 % bis 98 % Methylenchlorid und 2 % bis 5 % Isopropanol umfasst.

38. Verfahren nach einem der Ansprüche 29 bis 37, **dadurch gekennzeichnet, dass** in Schritt (e₆):
- die durch Chromatographie in Schritt (e₅) erhaltenen, Paclitaxel enthaltenden, angereicherten Fraktionen entsprechend ihrer Reinheit neu gruppiert, zur Trockne verdampft und dann erneut, in Methanol, zur Lösung gebracht werden, wobei das Volumen des zugegebenen Methanols so bemessen wird, dass ein Absorptionsvermögen erzielt wird, welches zwischen 1,0 bis 1,5 O.D. für den Paclitaxel entsprechenden Gipfel gemäß der HPLC-Analyse variiert; und
- das Paclitaxel dann zum dritten Mal, durch Zugabe von 2 bis 10 Volumina Wasser zu der Methanol-Lösung, rekristallisiert wird.

39. Verfahren nach Anspruch 38, **dadurch gekennzeichnet, dass** das Paclitaxel zum dritten Mal, durch Zugabe von 4 bis 7 Volumina Wasser zu der Methanol-Lösung, rekristallisiert wird.

40. Verfahren nach einem der Ansprüche 29 bis 39, **dadurch gekennzeichnet, dass** das in den Schritten (e₁), (e₂) und (e₃) verwendete flüchtige Lösemittel aus der Gruppe bestehend aus Aceton, leichten C1-C3-Alkoholen, Ethylacetat, Methylenchlorid und Mischungen dieser Lösemittel ausgewählt ist.

41. Verfahren nach einem der Ansprüche 29 bis 40, **dadurch gekennzeichnet, dass** in Schritt (e₆), nach der Kristallisation des Paclitaxels in Wasser, eine Mischung von Paclitaxel-Kristallen durch Filtern erhalten und dann getrocknet wird.

42. Verfahren nach einem der Ansprüche 1 bis 41, **dadurch gekennzeichnet, dass** das im gesamten Verfahren verwendete Wasser durch Entionisieren und/oder Destillieren gereinigt wird.

## Revendications

1. Procédé d'extraction et de purification du paclitaxel à partir d'une source naturelle de taxanes contenant le paclitaxel à extraire, ledit procédé comportant les étapes suivantes :
a) extraction, à l'aide d'un solvant organique, d'une matière première comportant du paclitaxel à partir de ladite source naturelle de taxanes;
b) traitement de ladite matière première dans un milieu basique ou acide pour obtenir une biomasse par précipitation, isolation de ladite biomasse et séchage de celle-ci ;
c) percoloration de la biomasse ainsi isolée par enlèvement de la résine et des pigments naturels contenus dans celle-ci, dissolution de ladite biomasse dans de l'acétone et puis ajout à celle-ci d'au moins un solvant.non polaire, jusqu'à ce qu'une phase huileuse enrichie en paclitaxel soit obtenue;
d) traitement de la biomasse contenue dans la phase huileuse enrichie en paclitaxel récupérée dans l'étape précédente, dans un milieu acide quand le traitement de l'étape b) a été effectué dans un milieu basique, ou dans un milieu basique quand le traitement de l'étape b) a été effectué dans un milieu acide, de façon à obtenir une autre biomasse, isolation de cet autre biomasse et séchage de celle-ci;
e) purification chromatographique effectuée au moins une fois de l'autre biomasse isolée obtenue dans l'étape précédente dans un solvant volatil du précipité isolé et cristallisation effectuée au moins une fois de la solution purifiée obtenue par chromatographie.

2. Procédé selon la revendication 1, **caractérisé en ce que** la source naturelle de taxanes contenant du paclitaxel est constituée de conifères choisis dans le groupe constitué par *Taxas brevifolia, Taxus baccara, Taxus canadensis, Taxus walichiana, Taxus yunnanensis, Taxus densiformis, Taxus hicksii, Taxus wardii, Taxus cuspidata, Taxus capitata, Taxus brownii*

3. Procédé selon la revendication 1 ou 2, **caractérisé en ce que** dans l'étape a), toutes les parties des conifères sont traitées.

4. Procédé selon l'une quelconque des revendications 1 à 2, **caractérisé en ce que**, dans l'étape a), des écorces des conifères sont traitées.

5. Procédé selon l'une quelconque des revendications 1 à 2, **caractérisé en ce que** dans l'étape a), des branches et des aiguilles des conifères sont simultanément traitées.

6. Procédé selon l'une quelconque des revendications 1 à 5, **caractérisé en ce que** dans l'étape a), le solvant organique est choisi dans le groupe constitué par les alcools, les hydrocarbures halogénés et les mélanges d'alcools et d'hydrocarbures halogénés.

7. Procédé selon la revendication 6, **caractérisé en ce que** dans l'étape a), le solvant est un mélange de méthanol et de dichlorométhane ou un mélange de méthanol et de trichlorométhane.

8. Procédé selon la revendication 6 ou 7, **caractérisé en ce que** l'alcool et l'hydrocarbure halogéné sont présent dans un rapport volumique compris entre 9:1 et 1:9.

9. Procédé selon la revendication 8, **caractérisé en ce que** le rapport volumique d'alcool par rapport à l'hydrocarbure halogéné est d'environ 1:1.

10. Procédé selon l'une quelconque des revendications 1 à 9, **caractérisé en ce que** dans l'étape b), la biomasse est précipité dans un milieu basique constitué d'une solution d'un sel basique choisi dans le groupe constitué par l'acétate de sodium, l'acétate de potassium, l'hydroxyde de sodium et le tris(hydroxyméthyl)aminométhane.

11. Procédé selon la revendication 10, **caractérisé en ce que** :
le sel basique est l'hydroxyde de sodium;
l'hydroxyde de sodium est utilisé dans une concentration comprise entre 10 mM et 80 mM par litre de solution; et
le pH de la solution est compris entre 8,5 et 11.

12. Procédé selon la revendication 10 et 11, **caractérisé en ce que** le pH de la solution est compris entre 9 et 10.

13. Procédé selon l'une quelconque des revendications 1 à 10, **caractérisé en ce que** dans l'étape b), la biomasse est précipitée dans un milieu acide constitué par une solution d'un acide minéral ou organique.

14. Procédé selon la revendication 13, **caractérisé en ce que** l'acide est choisi dans le groupe constitué par les acides chlorhydrique, acétique et citrique.

15. Procédé selon l'une quelconque des revendications 13 à 14, **caractérisé en ce que** la concentration en acide de ladite solution est comprise entre 10 mM et 50 mM par litre de solution.

16. Procédé selon la revendication 15, **caractérisé en ce que** la concentration en acide est comprise entre 20 mM et 30 mM par litre de solution.

17. Procédé selon l'une quelconque des revendications 13 à 16, **caractérisé en ce que** le pH de la solution est compris entre 2 et 4.

18. Procédé selon l'une quelconque des revendications 1 à 17, **caractérisé en ce que** dans l'étape b), la précipitation de la biomasse est effectuée dans une solution de ladite base ou dudit acide dans un rapport volumétrique de ladite solution par rapport à la matière première extraite dans l'étape a), compris entre 1 et 10.

19. Procédé selon l'une quelconque des revendications 1 à 18, **caractérisé en ce que** la biomasse précipitée est salée avant son isolation et séchage, par ajout de chlorure de sodium à une concentration comprise entre 25 et 200 g par litre de la solution utilisée pour la précipitation.

20. Procédé selon la revendication 19, **caractérisé en ce que** le chlorure de sodium est ajouté à une concentration comprise entre 50 et 100 g par litre de la solution utilisée pour la précipitation.

21. Procédé selon l'une quelconque des revendications 1 à 20, **caractérisé en ce que** la biomasse précipitée est séparée par filtration ou par centrifugation et puis séchée par ventilation ou par lyophilisation.

22. Procédé selon l'une quelconque des revendications 1 à 21, **caractérisé en ce que** le ou les solvant(s) non polaire(s) utilisé(s) dans l'étape c) est (sont) choisi(s) dans le groupe constitué par l'hexane ou l'heptane.

23. Procédé selon l'une quelconque des revendications 1 à 22, **caractérisé en ce que** dans l'étape c), la biomasse séchée obtenue dans l'étape b) est remise en solution par addition d'un volume d'acétone égal à 2 volumes de la matière première extraite dans l'étape a).

24. Procédé selon l'une quelconque des revendications 1 à 22, **caractérisé en ce que** la biomasse séchée obtenue à l'étape b) est remise en solution par ajout d'acétone puis ajout d'eau.

25. Procédé selon la revendication 24, **caractérisé en ce que** de l'eau est ajoutée dans un rapport de 2 à 10 volumes pour 100 volumes d'acétone ajouté.

26. Procédé selon l'une quelconque des revendications 1 à 25, **caractérisé en ce que** dans l'étape d):
- la phase enrichie en paclitaxel obtenue dans l'étape c) est évaporée à sec et remise en solution dans du méthanol, et
- ladite solution de méthanol est alors précipitée dans un milieu basique lorsque la première précipitation décrite dans l'étape b) a été réalisée dans un milieu acide, ou dans un milieu acide lorsque la première précipitation décrite dans l'étape b) a été réalisée en milieu basique,
- pour donner une biomasse contenant du paclitaxel, ledit paclitaxel étant sous forme d'un précipité.

27. Procédé selon l'une quelconque des revendications 1 à 26, **caractérisé en ce que** la biomasse obtenue dans l'étape d) est en outre salée avant son isolation et séchage par ajout de chlorure de sodium à une concentration comprise entre 30 et 200 g par litre de la solution utilisée pour la précipitation.

28. Procédé selon la revendication 27, **caractérisé en ce que** le chlorure de sodium est ajouté à une concentration comprise entre 50 et 100 g par litre de la solution utilisée pour la précipitation.

29. Procédé selon l'une quelconque des revendications 1 à 28, **caractérisée en ce que** l'étape e) comprend les sous-étapes suivantes:
(e1) une première purification chromatographique comprenant la dissolution du précipité isolé dans l'étape d) dans un solvant volatil, la préparation d'un mélange de la solution ainsi obtenue avec du gel de silice, le traitement dudit mélange sur une colonne chromatographique contenant du gel de silice, et récupération de fractions enrichies en paclitaxel;
(e2) une deuxième purification chromatographique comprenant l'évaporation à sec des fractions enrichies en paclitaxel récupérées dans l'étape précédente jusqu'à obtention d'un résidu, et la préparation d'un mélange par solubilisation dudit résidu dans un solvant volatil, ledit mélange étant repurifié par chromatographie dans les mêmes conditions que dans la sous-étape précédente pour fournir d'autres fractions enrichies en paclitaxel;
(e3) une première cristallisation comprenant l'évaporation à sec des autres fractions enrichies en paclitaxel recueillies dans la sous-étape précédente jusqu'à obtention d'un résidu, la préparation d'un mélange dudit résidu dans de l'acétone et la cristallisation du paclitaxel contenu dans le mélange avec un solvant non polaire;
(e4) une deuxième cristallisation comprenant la solubilisation des cristaux de paclitaxel obtenus dans la sous-étape précédente dans de l'acétone et la recristallisation du paclitaxel dans les mêmes conditions que dans la sous-étape précédente;
(e5) une troisième purification chromatographique comprenant la solubilisation des cristaux obtenus par recristallisation dans la sous-étape précédente dans un solvant volatil pour obtenir une soluition, la préparation d'un mélange de ladite solution avec du gel de silice et le traitement dudit mélange dans une colonne chromatographique contenant du gel de silice pour obtenir à l'aide d'un solvant d'élution, encore d'autres fractions enrichies en paclitaxel; et
(e6) une troisième cristallisation comprenant l'évaporation à sec des autres fractions enrichies en paclitaxel obtenues dans la sous-étape précédente jusqu'à l'obtention d'un résidu, la solubilisation du résidu dans un alcool, de l'acétone ou dans un mélange alcool-acétone pour obtenir un autre mélange, et la cristallisation du paclitaxel contenu dans l'autre mélange avec de l'eau.

30. Procédé selon la revendication 29, **caractérisé en ce que** dans l'étape (e1):
- la biomasse obtenue dans l'étape d) est remise en solution dans un solvant volatil;
- la solution obtenu est mélangée avec un gel de silice et séchée sous ventilation;
- le gel de silice recouvert de la biomasse est déposé sur une colonne chromatographique contenant du gel de silice; et
- la biomasse est alors purifiée avec un mélange d'élution comprenant de 30% à 40% d'acétone et de 60% à 70% d'hexane.

31. Procédé selon la revendication 30, **caractérisé en ce que** le mélange d'élution comprend environ 40% d'acétone et environ 60% d'hexane.

32. Procédé selon l'une quelconque des revendications 29 à 31, **caractérisé en ce que** dans l'étape (e2):
- les fractions enrichies contenant du paclitaxel obtenues par chromatographie à l'étape (e1), sont regroupées, évaporées à sec et remises en solution dans un solvant volatil;
- la solution obtenue est mélangée avec un gel de silice et séchée sous ventilation;
- le gel de silice recouvert de la biomasse est déposé sur une colonne de chromatographie contenant du gel de silice; et
- le paclitaxel est alors repurifié par un mélange de solvant organique comprenant de 30 à 40% d'acétone et de 60 à 70% d'hexane.

33. Procédé selon la revendication 32 **caractérisé en ce que** le mélange de solvant organique contient environ 40% d'acétone et 60% d'hexane.

34. Procédé selon l'une quelconque des revendications 29 à 31, **caractérisé en ce que** dans l'étape (e3):
- les fractions contenant du paclitaxel obtenues par chromatographie dans l'étape (e2), sont regroupées et évaporées à sec, et remises en solution dans l'acétone, la quantité d'acétone étant ajustée de façon à obtenir une absorbance de ladite solution ayant une valeur de 1,0 à 1,5 D.O., pour le pic correspondant au paclitaxel selon l'analyse par HPLC, et
- le paclitaxel est alors cristallisé en ajoutant de 3 à 4 volumes d'hexane dans la solution d'acétone.

35. Procédé selon l'une quelconque des revendications 29 à 34, **caractérisé en ce que** dans l'étape (e4):
- les cristaux obtenus dans l'étape (e3) sont séparés par filtration et remis en solution dans l'acétone, le volume d'acétone étant ajusté de façon à obtenir une absorbance de ladite solution variant de 1,0 à 1,5 D.O. pour le pic correspondant au paclitaxel selon l'analyse par HPLC; et
- le paclitaxel est alors recristallisé en ajoutant de 3 à 4 volumes d'hexane dans la solution d'acétone.

36. Procédé selon l'une quelconque des revendications 29 à 35, **caractérisé en ce que** dans l'étape (e5):
- les cristaux obtenus dans l'étape (e4) sont filtrés et remis en solution dans le chlorure de méthylène;
- la solution ainsi obtenue est alors mélangée avec un gel de silice et séchée sous ventilation;
- le gel de silice est recouvert de paclitaxel est déposé sur une colonne chromatographique contenant du gel de silice; et
- le paclitaxel est alors repurifié pour une troisième fois par un mélange d'élution à base de solvants organiques.

37. Procédé selon la revendication 36, **caractérisé en ce que** le mélange d'élution comprend de 95 à 98% de chlorure de méthylène et de 2 à 5% d'isopropanol.

38. Procédé selon l'une quelconque des revendications 29 à 37, **caractérisé en ce que** dans l'étape (e6):
- les fractions enrichies contenant du paclitaxel obtenues par chromatographie dans l'étape (e5) sont regroupées selon leur pureté, évaporées à sec et puis remise en solution dans le méthanol, le volume de méthanol étant ajouté de façon à obtenir une absorbance variant de 1,0 à 1,5 D.O. pour le pic correspondant au paclitaxel selon l'analyse par HPLC; et
- le paclitaxel est alors recristallisé pour la troisième fois par ajout de 2 à 10 volumes d'eau dans la solution de méthanol.

39. Procédé selon la revendication 50, **caractérisé en ce que** le paclitaxel est recristallisé pour la troisième fois par ajout de 4 à 7 volumes d'eau dans la solution de méthanol.

40. Procédé selon l'une quelconque des revendications 29 à 39, **caractérisé en ce que** le solvant volatil utilisé dans les étapes (e1), (e2) et (e3) est choisi dans le groupe constitué par l'acétone, les alcools légers en C1-C3, l'acétate d'éthyle, le chlorure de méthylène et les mélanges de ces solvants.

41. Procédé selon l'une quelconque des revendications 29 à 40, **caractérisé en ce que**, dans l'étape (e6), après cristallisation du paclitaxel dans l'eau, un mélange de cristaux de paclitaxel est obtenu après filtration puis séchage.

42. Procédé selon l'une quelconque des revendications 1 à 41, **caractérisé en ce que** l'eau utilisée dans l'ensemble du procédé est purifiée par désionisation et/ou par distillation.
